(19) Europäisches Patentamt
European Patent Office
Office européen des brevets

(11) Publication number: 0 212 260 A1

(12) EUROPEAN PATENT APPLICATION

(21) Application number: 86109786.3

(22) Date of filing: 16.07.86

(51) Int. Cl.[4]: C 07 D 499/68, C 07 D 499/14

(30) Priority: 17.07.85 JP 157363/85

(43) Date of publication of application: 04.03.87 Bulletin 87/10

(84) Designated Contracting States: DE FR GB NL SE

(71) Applicant: Yoshitomi Pharmaceutical Industries, Ltd., 35 Hiranomachi 3-chome Higashi-ku, Osaka-shi Osaka 541 (JP)

(72) Inventor: Araki, Kazuhiko, 1212-4, Aza-Azema Oaza-Kamiikenaga, Nakatsu-shi Oita (JP)
Inventor: Uemori, Satoru, 1828-3, Oaza-Shiroumaru, Buzen-shi Fukuoka (JP)
Inventor: Kuroda, Tsuyoshi, 1345, Nakatsu-shi, Oita (JP)
Inventor: Moriguchi, Akihiko, 98, Oaza-Ariyasu Shiidamachi, Chikujo-gun Fukuoka (JP)

(74) Representative: Vossius & Partner, Siebertstrasse 4 P.O. Box 86 07 67, D-8000 München 86 (DE)

(54) **Process for preparing a stable bacampicillin 6beta-Halopenicillanate.**

(57) A process for preparing a stable Bacampicillin 6β-halopenicillanate which comprises reacting Bacampicillin hydrochloride with an alkali metal salt or an amine salt of 6β-halopenicillanic acid under heating. The salt produced by the method of the present invention is remarkably stable against coloration and thermal decomposition in the long term storage.

EP 0 212 260 A1

VOSSIUS & PARTNER
PATENTANWÄLTE
EUROPEAN PATENT ATTORNEYS
SIEBERTSTRASSE 4 · 8000 MÜNCHEN 86 · PHONE: (089) 47 40 75
TELEX 529 453 VOPAT D TELEFAX (089) 47 20 01 (GR. II+III)

0212260

u. Z.: U 750 EP
Yoshitomi Pharmaceutical Industries,Ltd.
Japan

July 16, 1986

# PROCESS FOR PREPARING A STABLE BACAMPICILLIN · 6β-HALOPENICILLANATE

The present invention relates to a process for preparing a stable Bacampicillin · 6β-halopenicillanate.

It is known that 6β-halopenicillanic acid is a potent inhibitor of β-lactamase produced by various kinds of gram-positive and gram-negative bacteria, and further that the salt of Bacampicillin which is one of the orally administrable penicillin derivatives, with 6β-halopenicillanic acid protects Bacampicillin against inactivation with β-lactamase producing bacteria, expands antibacterial spectrum of Bacampicillin and increases antibacterial activity of Bacampicillin as disclosed in GB-A-2051046.

The above-mentioned GB-A-2051046 describes Bacampicillin · 6β-halopenicillanate, but its specification does not sufficiently disclose the process for the production of the salt to enable one skilled in the art to make the salt.

Since it is known that the reaction in the production of β-lactam antibiotics such as penicillin compounds is

generally carried out at as low a temperature as possible in order to avoid the decomposition of the compounds, it can be believed that the reaction of Bacampicillin hydrochloride with 6β-bromopenicillanic acid described in the prior art was also carried out below room temperature.

According to the present inventors' studies, the salt prepared in accordance with the prior art, however, was extremely unstable. Such salt was remarkably decomposed and the color of its appearance changed to brown in a stability test in an accelarated manner at 60 ℃ for 4 days or 7 days. These facts indicate that the Bacampicillin · 6β-bromopenicillanate prepared at room temperature or below unavoidably decomposes with coloration by being kept for a long term even at room temperature.

Such salt is, therefore, not suitable for the active ingredient of drugs.

Then, the present inventiors have investigated intensively in order to solve the above problems and considered that impurities derived from the salt of 6β-halopenicillanic acid as the starting material or related degradation products thereof are contaminated in the course of the production of the Bacampicillin · 6β-halopenicillanate and such contaminations cause the decomposition of the objective salt with coloration by

heat treatment.

As a result of intensive researches on various reaction conditions to remove such contamination in the production of the salt, the present inventors have found that a stable compound can be surprisingly obtained by carrying out the reaction under heating. Namely, the present inventors have found that a salt with a stable crystalline form and without contamination by the above-mentioned impurities or degradation products can be obtained by raising the reaction temperature from room temperature to 30-70℃.

This finding has led to the present invention.

The present invention relates to a process for preparing a stable Bacampicillin · 6β-halopenicillanate of the formula (I):

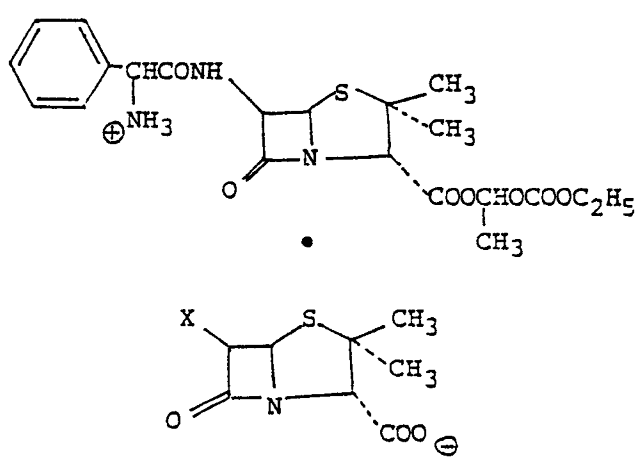

wherein X is bromine, iodine, chlorine or fluorine, which comprises reacting Bacampicillin hydrochloride with an

alkali metal salt or an amine salt of 6β-halopenicillanic acid of the formura (II):

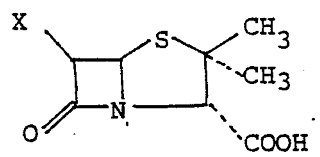

(II)

wherein X is as defined above, under heating.

In the process of the present invention, 6β-halopenicillanic acid includes 6β-bromo-, 6β-iodo-, 6β-chloro- and 6β-fluoropenicillanic acids, the alkali metal salt thereof includes, for example, lithium, sodium and potassium salts, and the amine salt thereof includes, for example, a salt with triethylamine, diethanolamine, triethanolamine, dicyclohexylamine, N-methylbenzylamine, N-ethylbenzylamine or the like. The most preferable salt are sodium and potassium salts.

The reaction is carried out at 30-70℃ (higher than room temperature), preferable at 30-50℃ for 0.5-2 hours in water, an aqueous solvent (e.g. 5-10% aqueous ethanol) or the like.

The following stability tests explain the effects of the present invention.

Stability of both Bacampicillin · 6β-bromopenicillanate produced by the process of the present invention and the known method was measured and the results are

summarized in the Table I and Table II.

As a degree of coloration, Hunter's color difference is measured by using a colorimeter (SM color computer; Model SM-2, manufactured by Suga Test Instruments, Japan), and contents of Bacampicillin and 6$\beta$-bromopenicillanic acid are determined according to High Performance Liquid Chromatography (HPLC) under the following conditions.

HPLC conditions:

Column: 20.0 cm, 4.0 mm i.d. stainless steel column packed with Nucleosil $7C_{18}$ (Chemco)

Eluent: Acetonitrile : 0.2M $KH_2PO_4$ (pH2.9) = 35 : 65

Flow: 1.0 mℓ/minute

Detector wavelength: 235 nm.

The test compounds employed are as follows:

A: Compound prepared by the known process (at room temperature)

1: Compound of Example 1 of the present invention

2: Compound of Example 2 of the present invention

3: Compound of Example 3 of the present invention

Table I

| Compound | Initial degree of yellow coloration | Storage conditions | | | |
|---|---|---|---|---|---|
| | | 60℃ for 4 days | | 60℃ for 7 days | |
| | | Degree of yellow coloration | ΔE | Degree of yellow coloration | ΔE |
| A | 8.3 | 62.5 | 30.5 | 62.3 | 30.2 |
| 1 | 7.1 | 11.6 | 3.1 | 13.2 | 3.9 |
| 2 | 5.4 | 10.0 | 2.5 | 11.8 | 3.4 |
| 3 | 7.7 | 12.4 | 2.7 | 14.1 | 3.9 |

ΔE: Color difference

Table II

| Compound | Contents (%) 60℃ for 7 days | |
|---|---|---|
| | Bacampicillin | 6β-bromopenicillanic acid |
| A | 94.6 | 87.4 |
| 1 | 101.5 | 100.1 |
| 2 | 100.5 | 97.8 |
| 3 | 101.0 | 98.9 |

It is indicated from the results shown in Table I and Table II that the Bacampicillin · 6β-bromopenicillanate produced at room temperature was considerably colored in appearance and the contents were remarkably decreased by heating at 60℃ for 4 and 7 days. On the other hand, the appearance and contents of the salt produced by the process of the present invention were not changed. In brief, the salts produced at 30-50℃ were stable against coloration and thermal decomposition, though the salt produced at 20℃ was extremely colored. The salts produced at 60℃ and 70℃ were slightly colored but salt produced at 80℃ or more was already colored to light-brown at the time of the production.

It can be understood from various viewpoints including the above-mentioned stability tests that a significantly stable Bacampicillin · 6β-halopenicillanate can be obtained by carrying out the reaction under heating at 30-70℃, preferably at 30-50℃.

Example I

To a solution of 128.52 g of Bacampicillin hydrochloride in 2400 mℓ of water was added dropwise a solution of 62.58 g of sodium 6β-bromopenicillanate in 600 mℓ of water under stirring and heating at 50℃ over 20 minutes. White crystals were immediately precipitated with the addition. After completion of the addition, the whole mixture was further stirred at the same temperature for 30 minutes. The hot mixture filtered to collect crystals. The crystals were washed with 500 mℓ of an aqueous solution of 5% ethyl acetate and dried under reduced pressure to give Bacampicillin · 6β-bromopenicillanate, melting at 120.7℃ with decomposition.

Example 2

To a solution of 21.42 g of Bacampicillin hydrochloride in 400 mℓ of 10% ethanol was added dropwise a solution of 10.43 g of sodium 6β-bromopenicillanate in 100 mℓ of 10% ethanol under stirring and heating at 50℃ over 20 minutes. The salt of Bacampicillin · 6β-bromopenicillanate was also obtained by the same manner as

described in the example 1.

Example 3

To a solution of 128.52 g of Bacampicillin hydrochloride in 2400 mℓ of 10% ethanol was added dropwise a solution of 62.58 g of sodium 6β-bromopenicillanate in 600 mℓ of 10% ethanol under stirring and heating at 30℃ over 20 minutes. The salt of Bacampicillin·6β-bromopenicillanate was also obtained by the same manner as described in the example 1.

Example 4

Bacampicillin 6β-chloropenicillanate can be obtained by reacting Bacampicillin hydrochloride with sodium 6β-chloropenicillanate at 50℃ in a similar manner described in the above examples.

Example 5

Bacampicillin 6β-iodopenicillanate can be obtained by reacting Bacampicillin hydrochloride with sodium 6β-iodopenicillanate at 50℃ in a similar manner described in the above examples.

# CLAIMS

1. A process for preparing a stable Bacampicillin · 6-β-halopenicillanate of the formula:

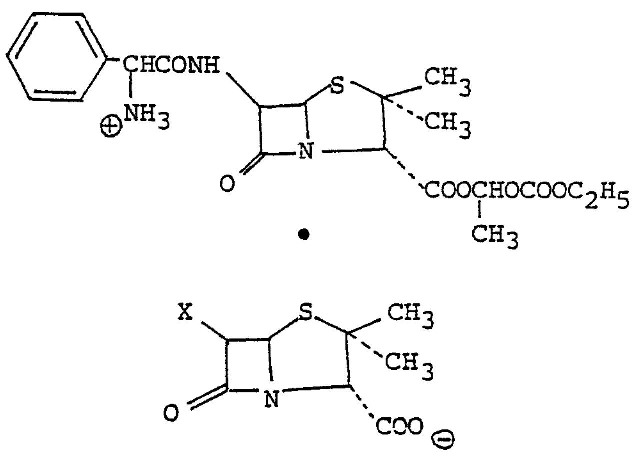

wherein X is bromine, iodine, chlorine or fluorine, which comprises reacting Bacampicillin hydrochloride with an alkali metal salt or an amine salt of 6β-halopenicillanic acid of the formula:

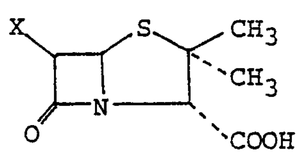

wherein X is as defined above, under heating.

2. The process of claim 1, said reaction is carried out at 30℃ to 70℃.

3. The process of claim 1, said reaction is carried out at 30℃ to 50℃.

European Patent Office

# EUROPEAN SEARCH REPORT

0212260

Application number

EP 86 10 9786

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | GB-A-2 051 046 (LEO PHARM.) * Page 14, example 22; page 16, example 29a; claims 1,7,25,26 * | 1 | C 07 D 499/68<br>C 07 D 499/14 |
| | --- | | |
| A | GB-A-2 087 236 (LEO PHARM.) * Pages 4,5, examples 8-13; claims * | 1 | |
| | ----- | | |

| TECHNICAL FIELDS SEARCHED (Int. Cl.4) |
|---|
| C 07 D 499/00<br>A 61 K 31/00 |

The present search report has been drawn up for all claims

| Place of search | Date of completion of the search | Examiner |
|---|---|---|
| THE HAGUE | 24-09-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82